# EUROPEAN PATENT APPLICATION

(11) **EP 2 258 855 A1**
(43) Date of publication of application: **08.12.2010**
(21) Application number: 09161403.2
(22) Date of filing: 28.05.2009
(51) Int. Cl.: C12N 15/81, C07K 14/39

(54) **Expression sequences**

(71) Applicant: Universität für Bodenkultur Wien, 1180 Wien (AT)
(72) Inventor: Mattanovich, Diethard, 1120 Vienna (AT); Gasser, Brigitte, 1050 Vienna (AT); Sauer, Michael, 1040 Vienna (AT); Maurer, Michael, 1030 Vienna (AT); Dragosits, Martin, 1030 Vienna (AT); Marx, Hans, 7072 Mörbisch (AT)
(74) Representative: Redl, Gerda

(57) **Abstract**

The invention relates to a regulatory sequence of a *Pichia pastoris* derived expression system capable of expressing the Epx1 protein that comprises the sequence of the mature protein of amino acid sequences SEQ ID No 1 or 2, which regulatory sequence is selected from the group consisting of a promoter, a signal sequence, a leader sequence and combinations thereof. The invention further relates to a vector and host cell comprising such regulatory sequences, and their use in producing a protein of interest.

## Description

The invention relates to regulatory elements of a *Pichia pastoris* (*P. pastoris*) expression system, and their use in a method to produce a protein of interest (POI).

Successful secretion of proteins has been accomplished both with prokaryotic and eukaryotic hosts. The most prominent examples are bacteria like *Escherichia coli,* yeasts like *Saccharomyces cerevisiae, Pichia pastoris* or *Hansenula polymorpha*, filamentous fungi like *Aspergillus awamori* or *Trichoderma reesei,* or mammalian cells like e.g. CHO cells. While the secretion of some proteins is readily achieved at high rates, many other proteins are only secreted at comparatively low levels.

The heterologous expression of a gene in a host organism requires a vector allowing stable transformation of the host organism. This vector has to provide the gene with a functional promoter adjacent to the 5' end of the coding sequence. The transcription is thereby regulated and initiated by this promoter sequence. Most promoters used up to date have been derived from genes that code for metabolic enzymes that are usually present at high concentrations in the cell.

EP0103409A2 discloses the use of yeast promoters associated with expression of specific enzymes in the glycolytic pathway, i.e. promoters involved in expression of pyruvate kinase, triosephosphate isomerase, phosphoglucose isomerase, phosphoglycerate mutase, hexokinase 1 and 2, glucokinase, phosphofructose kinase, aldolase and glycolytic regulation gene.

WO 97/44470 describes yeast promoters from *Yarrowia lipolytica* for the translation elongation factor 1 (TEF1) protein and for the ribosomal protein S7 that are suitable for heterologous expression of proteins in yeast, and EP1951877A1 describes the use of the *P. pastoris* TEF1 promoter for the production of heterologous proteins.

WO 2005/003310 provides methods for the expression of a coding sequence of interest in yeast using a promoter of the glyceraldehyde-3-phosphate dehydrogenase or phosphoglycerate mutase from oleaginous yeast *Yarrowia lipolytica*.

Promoter sequences derived from genes involved in the methanol metabolic pathway of *Pichia pastoris* are disclosed in US4808537 and US4855231 (alcohol oxidase AOX1, AOX2) and US6730499B1 (formaldehyde dehydrogenase FLD1). US020080153126A1 includes mutant promoter sequences based on the AOX1 promoter.

US 2008299616A1 introduces the regulatory sequences of the malate synthase (MLS1) gene for heterologous gene expression in *P. pastoris*, which is repressed in media containing glucose and derepressed under glucose starvation conditions or when acetate is present.

WO2008063302A2 describes the use of novel inducible promoters derived from ADH1 (alcohol dehydrogenase), ENO1 (enolase) and GUT1 genes of *P. pastoris* for the expression of heterologous proteins, CN1966688A the *P. pastoris* omega3- fatty acid dehydrogenase promoter sequence, and WO002007117062A1 the P. pastoris-derived auto-inducible NPS promoter, which is induced by phosphor limitation.

WO2008128701A2 provides novel regulatory sequences derived from P. *pastoris* for the expression of a gene of interest in yeasts.

The secretory pathway typically starts by translocation of transmembrane polypeptides and polypeptides intended for secretion into the lumen of the endoplasmic reticulum (ER). For that purpose, these proteins possess an amino-terminal precursing sequence comprising or consisting of a signal peptide and a secretion leader peptide. The signal peptide typically consists of 13 to 36 rather hydrophobic amino acids; no special consensus sequence has been identified yet. On the ER luminal side the signal peptide is removed by a signal peptidase. After successful folding of the nascent polypeptide by ER resident chaperones and foldases, the protein is further directed to exit the ER. This process may be supported by the presence of an N-terminal leader peptide, as it is present e.g. at the precurser of the *S. cerevisiae* mating factor alfa (MFA). The protein is then transported to the Golgi network and finally to the plasma membrane for secretion into the supernatant. The leader peptide is cleaved off the protein by (presumably) Golgi-resident proteases such as Kex2 protease of *S. cerevisiae*.

As the majority of yeasts do not secrete large amounts of endogenous proteins, and their extracellular proteomes are not extensively characterized so far, the number of available secretion sequences for the use in yeasts is limited. Therefore the fusion of the target protein to the mating factor alfa leader peptide (MFA) from *S. cerevisiae* was employed to drive secretory expression in many yeast species (including *Pichia, Kluyveromces, Zygosaccharomyces*). Unfortunately the proteolytic processing of the MFA by Kex2 protease often yields heterogeneous N-terminal amino acid residues in the product.

EP324274B1 describes improved expression and secretion of heterologous proteins in yeast employing truncated *S. cerevisiae* alfa-factor leader sequences, EP301669B1 the *Kluyveromyces* alfa-factor leader sequence for secretion of heterologous proteins.

Alternatively, the signal peptides of *S. cerevisiae* phosphatase (PHO5, DK3614), S. *cerevisiae* sucrose invertase (SUC, WO84/01153), and yeast aspartic protease 3 (YAP3, EP792367B1) were used for secretory expression in yeast. EP0438200 (A1) discloses the signal peptide sequence of *S. cerevisiae* SUC2 of for the expression in *P. pastoris*.

In the course of *P. pastoris* genome sequencing 54 different sequences were listed as predicted signal peptides (De Schutter et al. Nature Biotechnology doi: 10.1038/nbt.1544 2009).

It is desirable to provide alternative regulatory elements suitable for expressing a POI in a recombinant eukaryotic host cell, and methods to produce secreted proteins in eukaryotic cells which are simple and efficient, and could preferably lead to a homogenous N-terminus of the POI.

The object is solved by the subject matter as claimed.

The invention provides for new expression sequences, which are regulatory sequences of a *Pichia pastoris* derived expression system capable of expressing the Epx1 protein. The Exp1 protein protein comprises the sequence of the mature protein of amino acid sequences SEQ ID No 1 or 2, or homologous equivalents thereof. The regulatory sequence according to the invention is selected from the group consisting of a promoter, a signal sequence, a leader sequence and combinations thereof.

Preferred combinations are a combination of said signal sequence with said promoter and/or leader sequence.

A further preferred combination according to the invention is a combination of said promoter with said signal sequence and/or said leader sequence. According to a preferred embodiment of the invention, the sequence comprises the preferred promoter operatively linked to at least one of
- the preferred signal sequence, or
- the preferred leader sequence.

The sequence according to the invention preferably comprises or consists of a promoter consisting of a base sequence of at least 150bp having at least 70% homology with the base sequence of SEQ ID NO.: 7.

Another preferred sequence according to the invention comprises or consists of a signal sequence consisting of a base sequence encoding a peptide of at least 10 peptides, having at least 70% homology with the amino acid sequence of SEQ ID NO.: 3. Such a peptide is also called "signal peptide".

Another preferred sequence according to the invention comprises or consists of a leader sequence consisting of a base sequence encoding a peptide of at least 10 peptides, having at least 70% homology with the amino acid sequence of SEQ ID NO.: 4. Such a peptide is also called "leader peptide".

The sequence encoding at least part of a signal peptide and/ or at least part of a leader peptide is herein also called "precursing sequence".

The regulatory sequence according to the invention preferably comprises a cleavage site for the signal peptidase between a signal sequence and a leader sequence. Also preferred is a regulatory sequence according to the invention that comprises a cleavage site for a peptidase between a leader sequence and the N terminus of a POI. More preferred is a regulatory sequence according to the invention that contains both cleavage sites.

According to the invention there is also provided a vector comprising a regulatory sequence according to the invention, which is preferably suitable for integration into the genome of a host cell or for autonomous replication in a host cell.

Such a vector preferably comprises a gene encoding a POI, optionally with at least one processing site that provides for protease cleavage of said POI.

According to a specific embodiment of the invention there is provided a recombinant eukaryotic host cell comprising a regulatory sequence according to the invention or a vector according to the invention.

Such a host cell preferably is a fungal cell, preferably a yeast cell, or a higher eukaryotic cell, preferably a mammalian or a plant cell. In particular, a preferred host cell according to the invention is a cell of the Pichia genus, in particular a cell of a strain of *P. pastoris*.

A specific embodiment of the invention relates to a method of producing a POI in a eukaryotic cell, comprising:
- providing a host cell according to the invention containing a POI gene, and
- cultivating said host cell to express said POI.

Such a method preferably is employed to express a POI, which is a polypeptide selected from the group consisting of serum proteins, such as an immunoglobulin or serum albumin, enzymes, hormones, signalling molecules, matrix proteins, fragments or derivatives thereof.

Alternatively a POI is expressed, which mediates the production of a host cell metabolite.

The method according to the invention preferably provides for cultivation of the host cell in a cell culture, and said POI or metabolite is obtained as a secreted protein or metabolite, which is optionally purified from the cell culture supernatant.

According to the invention, the regulatory elements or sequences are preferably employed to modulate the expression of recombinant genes in host cells. When the Epx1 protein was purified, identified and characterized, it was surprising to obtain a new protein expressed by the host cell through a new expression system, which is greatly improved over comparable expression systems. Thus, a further embodiment of the invention refers to the Epx1 protein in the purified form, and a knock-out host cell, which comprises the Epx1 expression system, but would essentially not express the Epx1 protein, thus avoiding the endogenous Epx1 protein expression and contamination of product that is produced and secreted by the host cell. Preferably, the knock-out host cell is a *P. pastoris* host cell comprising a non-functional disruption of a regulatory sequence according to the invention and/or the sequence encoding the Epx1 protein.

### DETAILED DESCRIPTION OF THE INVENTION

"Biologically active fragment" of a protein shall mean a fragment of a protein that exerts a biological effect similar or comparable to the full length protein. Such fragments can be produced e.g. by amino- and carboxy- terminal deletions as well as by internal deletions.

The term "derivative" encompasses any combination of one or more compounds, e.g. polypeptides or proteins, and / or a fusion compound, in which any part of the compound may be fused at any position to one or more other polypeptides. A derivative may also be obtained by association or binding to other substances by various chemical techniques such as covalent coupling, electrostatic interaction, disulphide bonding etc. The other substances bound to the compound may be lipids, carbohydrates, nucleic acids, organic and inorganic molecules or any combination thereof (e.g. PEG, prodrugs or drugs). The term "derivative" would also comprise a homologous compound, also potentially containing non-natural or chemically modified amino acids, and fragments of compounds.

"Expression vectors" or "vectors" used herein are defined as DNA sequences that are required for the transcription of cloned recombinant nucleotide sequences, i.e. of recombinant genes and the translation of their mRNA in a suitable host organism. Such expression vectors usually comprise an origin for autonomous replication in the host cells, selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together.

The terms "plasmid" and "vector" as used herein include autonomously replicating nucleotide sequences as well as genome integrating nucleotide sequences.

The term "eukaryotic host" shall mean any eukaryotic cell or organism, which may be cultivated to express a POI or a host cell metabolite. It is well understood that the term does not include human beings.

The term "Epx1" shall mean a polypeptide of *P. pastoris* with homology to a protein that is characterized by the mature polypeptide sequence of SEQ ID No 1 or 2, which are encoding the same type of polypeptide, however originating from different *P. pastoris* strains. The Epx1 protein is preferably characterized upon expression by *P. pastoris* host organisms, such as DSMZ70382, or the Invitrogen strains X-33, GS115, KM71 and SMD1168. In particular the term includes any dimers or derivatives thereof, including a dimer structure with a molecular weight of about 60kD. As used herein the term Epx1 is used interchangeably for the gene and the gene product, including at least the mature protein, optionally together with at least part of the precursing sequences. Sequence informations to this polypeptide is provided below. The term shall encompass the full-length protein or biologically active fragments thereof, as well as functional homologues.

The term "functionally equivalent" or "variant" of a polypeptide or a nucleotide sequence as used herein means a sequence resulting from modification of this sequence by insertion, deletion or substitution of one or more amino acids or nucleotides within the sequence or at either or both of the distal ends of the sequence, and which modification does not affect (in particular impair) the activity of this sequence.

The term "gene product" or "product of a gene" is the biochemical material, either RNA or protein, resulting from expression of a gene, such as the Epx1 gene.

As used herein, a "homologue" or "functional homologue" of a polypeptide shall mean that polypeptides have the same or conserved residues at a corresponding position in their primary, secondary or tertiary structure. The term also extends to two or more nucleotide sequences encoding homologous polypeptides. In particular, homologous compounds usually have at least about 50% amino acid sequence identity with regard to a full-length native sequence or any fragment thereof. Preferably, a homologous compound will have at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity to a native compound, or any other specifically defined fragment of a full-length compound. When the function as a regulatory element is proven with such a homologue, the homologue is called "functional homologue" of said regulatory element.

The term "homologous nucleotide sequences" as used herein refers to nucleotide sequences which are related but not identical in their nucleotide sequence with the contemplated nucleotide sequence, and perform essentially the same function. These are also meant to encompass variations in its nucleotide composition including variations due to the degeneracy of the genetic code, whereby the nucleotide sequence performs essentially the same function.

The term "host cell" or "hosts cell line" refers to a microorganism, used for expression of a recombinant gene to produce polypeptides or metabolites mediated by such polypeptides. A host cell clone of cultivated host cells that have proliferated is commonly understood to be a host cell line. A production host cell line is commonly understood to be a cell line ready-to-use for cultivation in a bioreactor to obtain the gene product in a production method.

The term "operably linked" as used herein refers to the association of nucleotide sequences on a single nucleic acid molecule, e.g. a vector, in a way such that the function of one or more nucleotide sequences is affected by at least one other nucleotide sequence present on said nucleic acid molecule. For example, a promoter is operably linked with a coding sequence of a recombinant gene, when it is capable of effecting the expression of that coding sequence.

"Percent (%) amino acid sequence identity" with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

The term "polypeptide" refers to a protein or peptide that contains two or more amino acids, typically at least 3, preferably at least 20, more preferred at least 30, more preferred at least 50 amino acids. The term also refers to higher molecular weight polypeptides, such as proteins. Hereinafter the terms "polypeptide" and "protein" are interchangeably used.

"Promoter" as used herein refers to a DNA sequence capable of controlling the expression of a coding sequence or functional RNA. Promoter activity may be assessed by its transcriptional efficiency. This may be determined directly by measurement of the amount of mRNA transcription from the promoter, e.g. by Northern Blotting or indirectly by measurement of the amount of gene product expressed from the promoter.

The term "protein of interest (POI)" as used herein refers to a protein that is produced by means of recombinant technology in a host cell. More specifically, the protein may either be a polypeptide not naturally occurring in the host cell, i.e. a heterologous protein, or else may be native to the host cell, i.e. a homologous protein to the host cell, but is produced, for example, by transformation with a self replicating vector containing the nucleic acid sequence encoding the POI, or upon integration by recombinant techniques of one or more copies of the nucleic acid sequence encoding the POI into the genome of the host cell, or by recombinant modification of one or more regulatory sequences controlling the expression of the gene encoding the POI, e.g. of the promoter sequence. In some cases the term POI as used herein also refers to any metabolite product by the host cell as mediated by the recombinantly expressed protein.

The term "regulatory sequence" or "regulatory element" as used herein shall refer to a region of a nucleotide sequence that regulates the expression of genes in a cell. A regulatory element may be located in the promoter region 5' to the gene it controls, in an intron, or in the 3' untranslated region, or in a transcription region, when the transcripts modulate the expression or secretion of a polypeptide. Thus, the term shall in particular refer to a promoter, enhancer, silencer, response element, signal sequence or leader sequence.

The term "signal sequence" as used herein shall refer to a signal peptide, which is usually a short (3-60 amino acids long) peptide chain that directs the transport of a protein. Signal peptides may also be called targeting signals, signal sequences, transit peptides, or localization signals. Targeting signals are N-terminal sequences, which direct the protein (which is synthesized in the cytosol) to certain organelles such as the nucleus, mitochondrial matrix, endoplasmic reticulum, chloroplast, apoplast and peroxisome. A signal peptide induces an expressed protein to be transported outside the plasma membrane, thereby making it easy to separate and purify the heterologous protein. Generally, a membrane protein or a secretory protein which is transported into the periplasm space or outside of the cell comprises such an N-terminal sequence. Some signal peptides are cleaved from the protein by signal peptidase after the proteins are transported.

The term "leader sequence" as used herein shall refer to a coding sequence of a precursor polypeptide which is located N-terminal to the sequence of a mature polypeptide. The leader sequence usually comprises a processing site that provides for the cleavage of said leader from said precursor polypeptide. The precursor may be encoded by a nucleotide sequence which is comprised of a signal sequence, followed by a leader region, also called "pro"region, which is then linked to the sequence encoding the mature polypeptide. Thus, it is understood that the signal sequence and/or and the leader sequence, are "precursing sequences" as used according to the invention.

It was surprising to identify and characterize a new polypeptide of *P. pastoris* that was naturally overexpressed in some cases of recombinant protein expression. Epx1 was obtained as a secreted protein in the cell culture, and the first time purified and characterized. Surprisingly, the expression system, which is employed by nature to express the Epx1 polypeptide, is a unique, but versatile one that could be used to express high yields of a recombinant POI or host cell metabolite mediated by a recombinant protein. Either of the regulatory elements, such as the promoter, the signal sequence and the leader sequence are ideally used alone, in combination with conventional regulatory elements or in combination with each other, to provide constructs for recombinant protein production at high yields.

According to preferred mode of the invention the regulatory sequence according to the invention comprises a promoter having the base sequence of SEQ ID No 7, or functional homologues thereof.

According to another preferred mode of the invention the regulatory sequence according to the invention comprises a signal sequence having the amino acid sequence of SEQ ID No 3, or functional homologues thereof.

According to another preferred mode of the invention the regulatory sequence according to the invention comprises a leader sequence having the amino acid sequence of SEQ ID No 4, or functional homologues thereof.

The relevant regulatory sequences can be modified to be highly active in the host cell according to the invention. It was found that such regulatory sequences according to the invention, which have at least 70% homology to the native sequences can preferably be used as functionally equivalents of the native ones having the respective promoter activity or function as a signal sequence or a leader sequence. Preferably the homology is at least 80%, more preferably at least 90%, most preferably the identical sequence, or a relevant part thereof may be used according to the invention to support recombinant protein production.

Significant sequence activity may be obtained with only part of the regulatory sequences as illustrated by the respective SEQ ID numbers. While the signal peptide or the leader peptide preferably comprises at least 10 consecutive amino acids, more preferably at least 12, 14, 16 or 18 amino acids, the preferred part of the promoter sequence consists of at least 150 consecutive bases or bp, more preferred at least 200 bp, at least 300 bp, at least 400 bp, at least 500 bp, at least 600 bp, at least 700 bp, at least 800 bp or at least 900 bp. Preferably the 3' end of the promoter region is contained in the preferred part of the promoter sequence.

In particular, the combination of the preferred signal sequence with the preferred leader sequence is preferred, which has at least 60% of the full length sequence, preferably at least 70%, more preferred at least 80% or 90%.

To prove the function of the relevant sequences, expression vectors comprising one or more of the regulatory elements may be constructed to drive expression of a POI, and the expressed yield is compared to constructs with conventional regulatory elements. A detailed description of the experimental procedure can be found in the examples below. The identified genes were amplified by PCR from *P. pastoris* using specific nucleotide primers, cloned into a yeast expression vector and transformed into a strain of *P. pastoris* for high level production of various different POI, optionally in the secreted form. To estimate the effect of the regulatory sequences according to the invention on the amount of secreted recombinant POI, the obtained *P. pastoris* strains according to the invention may be cultured in shake flask experiments and fedbatch or chemostat fermentations in comparison with strains comprising conventional regulatory elements. In particular, the choice of the promoter has a great impact on the recombinant protein production.

By means of the inventive regulatory sequences the method according to the invention preferably not only provides for an increased production by an enhanced expression, but also to an increased secretion of the POI in a eukaryotic cell. An increase in secretion of the POI is determined on the basis of a comparison of its secretion yield in the presence of the regulatory sequence, in particular the signal sequence, that increases protein secretion as compared to a conventional element.

Preferably the N-terminus of the secreted POI is identical to the native N terminal sequence of the POI.

The POI can be any eukaryotic, prokaryotic or synthetic polypeptide. It can be a naturally secreted protein or an intracellular protein, i.e. a protein which is not naturally secreted. The present invention also provides for the recombinant production of functional homologues, functional equivalent variants, derivatives and biologically active fragments of naturally secreted or not naturally secreted proteins. Functional homologues are preferably identical with or correspond to and have the functional characteristics of a sequence.

A secreted POI referred to herein may be, but is not limited to, a protein suitable as a biopharmaceutical substance like an antibody or antibody fragment, growth factor, hormone, enzyme, vaccine, or a protein which can be used for industrial application like e.g. an enzyme.

The POI is preferably a heterologous recombinant polypeptide or protein, which may advantageously be produced in a eukaryotic cell, preferably a yeast cell, preferably as secreted proteins. Examples of preferably produced proteins are immunoglobulins, immunoglobulin fragments, aprotinin, tissue factor pathway inhibitor or other protease inhibitors, and insulin or insulin precursors, insulin analogues, growth hormones, interleukins, tissue plasminogen activator, transforming growth factor a or b, glucagon, glucagon-like peptide 1 (GLP-1), glucagon-like peptide 2 (GLP-2), GRPP, Factor VII, Factor VIII, Factor XIII, platelet-derived growth factor1, serum albumin, enzymes, such as lipases or proteases, or a functional analogue of any one of these proteins. In the present context, the term "functional analogue" is meant to indicate a polypeptide with a similar function as the native protein. The polypeptide may be structurally similar to the native protein and may be derived from the native protein by addition of one or more amino acids to either or both the C- and N-terminal end or the side-chain of the native protein, substitution of one or more amino acids at one or a number of different sites in the native amino acid sequence, deletion of one or more amino acids at either or both ends of the native protein or at one or several sites in the amino acid sequence, or insertion of one or more amino acids at one or more sites in the native amino acid sequence. Such modifications are well known for several of the proteins mentioned above.

A POI can also be selected from substrates, enzymes, inhibitors or cofactors that provide for biochemical reactions in the host cell, with the aim to obtain the product of said biochemical reaction or a cascade of several reactions, e.g. to obtain a metabolite of the host cell. Examplary products can be vitamins, such as riboflavin, organic acids, and alcohols, which can be obtained with increased yields following the expression of a recombinant protein or a POI according to the invention.

In general, the host cell, which expresses a recombinant product, can be any eukaryotic cell suitable for recombinant expression of a POI.

Examples of preferred yeast cells used as host cells according to the invention include but are not limited to the *Saccharomyces* genus (e.g. *Saccharomyces cerevisiae),* the *Pichia* genus (e.g. *P. pastoris, or P. methanolica*), the *Komagataella* genus (*K. pastoris, K. pseudopastoris* or *K. phaffii*), *Hansenula polymorpha* or *Kluyveromyces lactis*.

Newer literature divides and renames *Pichia pastoris* into *Komagataella pastoris, Komagataella phaffii* and *Komagataella pseudopastoris*. Herein *Pichia pastoris* is used synonymously for all, *Komagataella pastoris, Komagataella phaffii* and *Komagataella pseudopastoris*.

The yeast producer organism preferably used according to the invention may be any suitable yeast organism which, on cultivation, produces large amounts of the heterologous protein or polypeptide in question. Preferred examples of suitable yeast organisms are strains selected from the yeast species *Saccharomyces cerevisiae, Saccharomyces kluyveri, Schizosaccharomyces pombe, Saccharomyces uvarum, Kluyveromyces lactis, Hansenula polymorpha, Pichia pastoris, Pichia methanolica, Pichia kluyveri, Yarrowia lipolytica, Candida* sp., *Candida utilis, Candida cacaoi, Geotrichum* sp., and *Geotrichum fermentans*.

The most preferred yeast host cells are derived from methylotrophic yeast, such as from *Pichia* or *Komagataella*, e.g. *Pichia pastoris,* or *Komoagataella pastoris*, or *K. phaffii*, or *K. pseudopastoris*. Examples of the host include yeasts such as *P. pastoris*. Examples of *P. pastoris* strains include CBS 704 (=NRRL Y-1603 = DSMZ 70382), CBS 2612 (=NRRL Y-7556), CBS 7435 (=NRRL Y-11430), CBS 9173-9189, and DSMZ 70877 (German Collection of Microorganisms and Cell Cultures), but also strains from Invitrogen, such as X-33, GS115, KM71 and SMD1168. Examples of S. *cerevisiae* strains include W303, CEN.PK and the BY-series (EUROSCARF collection). All of the strains described above have been successfully used to produce transformants and express heterologous genes.

In general, the proteins of interest referred to herein may be produced by methods of recombinant expression well known to a person skilled in the art.

It is understood that the methods disclosed herein may further include cultivating said recombinant host cells under conditions permitting the expression of the POI in the secreted form. A secreted, recombinantly produced POI or a host cell metabolite can then be isolated from the cell culture medium and further purified by techniques well known to a person skilled in the art.

The nucleotide sequences that could be used as regulatory sequences according to the invention, which would provide for an improved recombinant protein production, can be obtained from a variety of sources. The origin of a promoter or a precursing sequence according to the invention is preferably from a yeast cell, most preferably from methylotrophic yeast such as from the Pichia genus. It is contemplated that a series of yeast cells, in particular Pichia strains, may be suitable to obtain respective regulatory sequences that are responsible for the Epx1 protein production, or respective analogues in different species. A preferred Pichia host cell according to the invention, such as a *P. pastoris* host cell, contains the heterologous regulatory sequences, which are preferably derived from a *P. pastoris* or *S. cerevisiae* strain, different from the production host. In another specific embodiment the host cell according to the invention comprises a recombinant nucleotide sequence with the regulatory sequences originating from the same strain as the host cell.

For example, a nucleotide sequence encoding one of the regulatory sequences according to the invention may be derived from yeast, such as a *S. cerevisiae* strain, and be used to express a POI in a yeast, such as a *S. cerevisiae* producer host cell line. A specifically preferred embodiment relates to a recombinant nucleotide sequence encoding a regulatory element originating from *P. pastoris* for use in a method to produce a recombinant POI in a *P. pastoris* producer host cell line. The homologous origin of the nucleotide sequence facilitates its incorporation into the host cell of the same genus, thus enabling stable production of a POI, possibly with increased yields in industrial manufacturing processes. Also, functional equivalent nucleotide sequences from other suitable yeasts or other fungi or from other organisms such as vertebrates or plants can be used.

According to the invention it is also possible to provide a wildcard vector or host cell according to the invention, which is comprising at least one of the regulatory sequences, and which is ready to incorporate a gene of interest encoding a POI. The wildcard cell line is thus a preformed host cell line, which is characerized for its expression capacity. This follows an innovative "wildcard" strategy for the generation of producer cell lines, also called expression host cell line, for the production of biopharmaceuticals, e.g. using site-specific recombinase-mediated cassette exchange. Such a new host cell facilitates the cloning of a gene of interest (GOI), e.g. into predetermined genomic expression hot spots within days in order to get reproducible, highly efficient production cell-lines.

Appropriate expression vectors comprise regulatory sequences suitable for expression of DNA encoding a heterologous polypeptide or protein in a eukaryotic host cell. Examples of regulatory sequences include promoters, operators, and enhancers, ribosomal binding sites, and sequences that control transcription and translation initiation and termination. According to the invention, at least one of the promoter, signal sequence or leader sequence is used as regulatory sequence. The regulatory sequences may be operably linked to the DNA sequence to be expressed. For example, a promoter sequence is said to be operably linked to a coding sequence, if the promotor controls the transcription of the coding sequence.

According to the invention it is preferred to provide a *P. pastoris* host comprising a regulatory sequence according to the invention operably linked to the nucleotide sequence coding for the POI.

According to a preferred embodiment the method according to the invention employs a recombinant nucleotide sequence encoding the POI, which is provided on a plasmid suitable for integration into the genome of the host cell, in a single copy or in multiple copies per cell. The recombinant nucleotide sequence encoding the POI may also be provided on an autonomously replicating plasmid in a single copy or in multiple copies per cell.

The preferred method according to the invention employs a plasmid, which is a eukaryotic expression vector, preferably a yeast expression vector. Expression vectors may include but are not limited to cloning vectors, modified cloning vectors and specifically designed plasmids. The preferred expression vector as used in the invention may be any expression vector suitable for expression of a recombinant gene in a host cell and is selected depending on the host organism. The recombinant expression vector may be any vector which is capable of replicating in or integrating into the genome of the host organisms, also called host vector, such as a yeast vector, which carries a DNA construct according to the invention. A preferred yeast expression vector is for expression in yeast selected from the group consisting of methylotrophic yeasts represented by the genera Hansenula, Pichia, Candida and Torulopsis.

In the present invention, it is preferred to use plasmids derived from pPICZ, pGAPZ, pPIC9, pPICZalfa, pGAPZalfa, pPIC9K, pGAPHis or pPUZZLE as the vector.

According to a preferred embodiment of the present invention, a recombinant construct is obtained by ligating the relevant genes into a vector. These genes can be stably integrated into the host cell genome by transforming the host cell using such vectors. The polypeptides encoded by the genes can be produced using the recombinant host cell line by culturing a transformant, thus obtained in an appropriate medium, isolating the expressed POI from the culture, and purifying it by a method appropriate for the expressed product, in particular to separate the POI from contaminating proteins.

Several different approaches for the POI expression and secretion in the eukaryotic host cell are preferred. Proteins are expressed, processed and secreted by transforming the eukaryotic organism with an expression vector harbouring DNA encoding the desired protein and at least one of the regulatory elements according to the invention, preparing a culture of the transformed organism, growing the culture and recovering the protein from the culture medium. The employed signal peptide may be the signal peptide according to the invention or an alternative one, e.g. a heterologous signal peptide or a hybrid of a native and a heterologous signal peptide. The function of the signal peptide is to allow the heterologous protein to be secreted to enter the endoplasmatic reticulum. The signal peptide is normally cleaved off in the course of this process. The signal peptide may be heterologous or homologous to the host organism producing the protein.

In a preferred aspect the invention relates to such a method, wherein the expression vector comprises a leader sequence effective to cause expression and secretion of the POI from the host cell. The presence of such a secretion leader sequence in the expression vector is required when the POI intended for recombinant expression and secretion is a protein which is not naturally secreted and therefore lacks a natural secretion leader sequence, or its nucleotide sequence has been cloned without its natural secretion leader sequence. The secretion leader sequence may be the leader sequence according to the invention or an alternative one, e.g. originating from yeast α-factor such as MFα of *S. cerevisiae*, or yeast phosphatase, from mammalian or plant source, or others. The selection of the appropriate secretion leader sequence is apparent to a skilled person.

The secretion leader sequence can be fused to the nucleotide sequence encoding a POI intended for recombinant expression by conventional cloning techniques known to a skilled person. In preferred embodiments, the nucleotide sequence of the POI is fused to a secretion signal, i. e. a peptide that targets protein to the secretory pathway where the signal peptide is cleaved and the protein released in the medium, for example alfa-factor, PHO or AGA-2.

The promoter may be a promoter according to the invention or any other DNA sequence which shows transcriptional activity in the host cell and may be derived from genes encoding proteins either homologous or heterologous to the host. The promoter is preferably derived from a gene encoding a protein homologous to the host cell.

A preferred method according to the invention refers to the increase of the secretion of a POI in a eukaryotic cell, wherein the gene encoding the POI is controlled by a promoter sequence which is a 1000 bp fragment from the 5'-non coding region of the Epx1 gene of *Pichia pastoris* corresponding to SEQ ID NO 1, or a functionally equivalent homologue thereof and the host cell is a cell of the genus *Komagataella,* in particular a cell of a strain of *K. pastoris, K. pseudopastoris* or *K. phaffii.*

By selecting the suitable promoter sequence according to the invention, optionally in combination with further preferred regulatory sequences, it is possible to provide for, under comparable conditions, at least the same, or at least about a 1.5-fold, or at least about 2-fold, or at least about a 4-fold, 7-fold, 10-fold, or at least up to about a 15-fold activity as represented by the promoter activity or regulated by the promoter activity relative to a GAP promoter isolated from *P. pastoris*.

If the cellular POI is a homologous protein to the host cell, i.e. a protein which is naturally occurring in the host cell, the expression of the POI in the host cell may be modulated by the exchange of its native promoter sequence with a the promoter sequence according to the invention.

This purpose may be achieved e.g. by transformation of a host cell with a recombinant DNA molecule comprising homologous sequences of the target gene to allow site specific recombination, the promoter sequence and a selective marker suitable for the host cell. The site specific recombination shall take place in order to operably link the promoter sequence with the nucleotide sequence encoding the POI. This results in the expression of the POI from the promoter sequence according to the invention instead of from the native promoter sequence.

In a specifically preferred embodiment of the invention the promoter sequence has an increased promoter activity relative to the native promoter sequence of the POI.

Depending on the problem to be solved the selected promoter may have either an increased promoter activity relative to the native promoter sequence leading to an increased expression of a POI in the host cell or may have a decreased promoter activity relative to the native promoter sequence leading to a reduced expression of a POI in the host cell. Thus, in an alternatively preferred embodiment of the invention the promoter sequence has a decreased promoter activity relative to the native promoter sequence of the POI, e.g. a weak promoter to specifically downregulate a regulatory gene.

To allow expression of a recombinant nucleotide sequence in a host cell, the expression vector may provide the recombinant nucleotide sequence with a functional promoter adjacent to the 5' end of the coding sequence, e.g. upstream from the signal peptide gene. The transcription is thereby regulated and initiated by this promoter sequence.

Suitable promoter sequences for use with mammalian host cells may include but are not limited to promoters obtained from the genomes of viruses, heterologous mammalian promoters, e.g. the actin promoter or an immunoglobulin promoter, and heat shock protein promoters. The promoter is not limited to any particular species provided that they can function in eukaryotic host cells and in particular in yeast.

Further suitable promoter sequences for use with yeast host cells may include but are not limited to promoters obtained from genes that code for metabolic enzymes which are known to be present at high concentration in the cell, e.g. glycolytic enzymes like triosephosphate isomerase (TPI), phosphoglycerate kinase (PGK), glyceraldehyde-3-phosphate dehydrogenase (GAPDH), alcohol oxidase (AOX), lactase (LAC) and galactosidase (GAL).

Preferred examples of suitable promoters are the yeast promoters, which contains a DNA sequence that functions as a promoter for gene transcription in yeast cells. Preferred examples are *S. cerevisiae* Mal, TPI, CUP, ADH or PGK promoters, or the *P. pastoris* glucose-6-phosphate isomerase promoter (PPGI), the 3-phosphoglycerate kinase promoter (PPGK) or glycerol aldehyde phosphate dehydrogenase promoter PGAP, the alcohol oxidase promoter (PAOX), formaldehyde dehydrogenase promoter (PFLD), isocitrate lyase promoter(PICL), translation elongation factor promoter (PTEF), and the promoters of P. pastoris enolase 1 (PENO1), triose phosphate isomerase (PTPI), alpha-ketoisocaproate decarboxylase (PTHI), ribosomal subunit proteins (PRPS2, PRPS7, PRPS31, PRPL1), heat shock protein family members (PSSA1, PHSP90, PKAR2), 6-Phosphogluconate dehydrogenase (PGND1), phosphoglycerate mutase (PGPM1), transketolase (PTKL1), phosphatidylinositol synthase (PPIS1), ferro-O2-oxidoreductase (PFET3), high affinity iron permease (PFTR1), repressible alkaline phosphatise (PPHO8), N-myristoyl transferase (PNMT1), pheromone response transcription factor (PMCM1), ubiquitin (PUBI4), single-stranded DNA endonuclease (PRAD2) and the promoter of the major ADP/ATP carrier of the mitochondrial inner membrane (PPET9).

In a preferred expression system the promoter is an inducible or a constitutive promoter. The promoter can be an endogenous promoter or heterologous to the host cell.

The preferred expression system makes use of a combination of the regulatory sequences according to the invention. Thus, it is preferred to provide for a promoter operatively linked to a signal sequence, both derived from the Epx1 expression system, followed by a leader sequence or not.

Secreted proteins generally are initially expressed as precursors bearing an N-terminal signal or leader peptide. Signal peptides generally contain a positively charged N-terminus followed by a hydrophobic core, followed by a recognition site for an enzyme known as signal peptidase. This enzyme cleaves the signal peptide from the protein during translocation into the ER. The protein is transported from the ER to the Golgi apparatus, and then follows one of a number of routes in the secretory pathway, depending on the nature of the protein. The protein may be secreted into the culture medium or may be retained on the cell surface, for example. Certain receptors that comprise extracellular, transmembrane, and cytoplasmic domains are examples of proteins that may be retained on the cell membrane, with only the extracellular domain located outside the cell.

When a signal sequence would not be necessary for the protein production, the promoter is advantegously operatively linked to a leader sequence or a sequence coding for the native polypeptide without a signal sequence.

When the signal sequence according to the invention is followed by a leader sequence according to the invention within the precursing sequence, it is preferred to provide for a cleavage site for the signal peptidase to enable expression of the proform of the polypeptide. Such as cleavage site can be derived from the precursing sequence of the Epx1 polypeptide. Alternative cleavage sites, however, can be employed by the skilled artisan.

When the construct provides for the expression of the precursor of a polypeptide, it is preferred to include a processing site between the precursing sequences and the sequence encoding the mature polypeptide. Thereby the cleavage of the precursing sequence is possible, through protease cleavage. A suitable cleavage site can be derived from the precursing sequence of the Epx1 polypeptide. Alternative cleavage sites, however, can be employed by the skilled artisan.

The DNA sequence encoding the POI may also be operably connected to a suitable terminator sequence, for example AOX1 (alcohol oxidase) terminator, CYC1 (cytochrome c) terminator, TEF (translation elongation factor) terminator.

Expression vectors may comprise one or more phenotypic selectable markers, e.g. a gene encoding a protein that confers antibiotic resistance or that supplies an autotrophic requirement. Yeast vectors commonly contain an origin of replication from a yeast plasmid, an autonomously replicating sequence (ARS), or alternatively, a sequence used for integration into the host genome, a promoter region, sequences for polyadenylation, sequences for transcription termination, and a selectable marker.

The procedures used to ligate the DNA sequences, e.g. coding for the precursing sequence and/or the POI, the promoter and the terminator, respectively, and to insert them into suitable vectors containing the information necessary for integration or host replication, are well known to persons skilled in the art, e.g. described by J. Sambrook et al., "Molecular Cloning 2nd ed.", Cold Spring Harbor Laboratory Press (1989).

It will be understood that the vector, which uses the regulatory elements according to the invention and/or the POI as an integration target, may be constructed either by first preparing a DNA construct containing the entire DNA sequence coding for the regulatory elements and/or the POI and subsequently inserting this fragment into a suitable expression vector, or by sequentially inserting DNA fragments containing genetic information for the individual elements, such as the signal, leader or heterologous protein, followed by ligation.

Also multicloning vectors, which are vectors having a multicloning site, can be used according to the invention, wherein a desired heterologous gene can be incorporated at a multicloning site to provide an expression vector. In expression vectors, the promoter is placed upstream of the gene of the POI and regulates the expression of the gene. In the case of multicloning vectors, because the gene of the POI is introduced at the multicloning site, the promoter is placed upstream of the multicloning site.

The DNA construct as provided to obtain a recombinant host cell according to the invention may be prepared synthetically by established standard methods, e.g. the phosphoramidite method. The DNA construct may also be of genomic or cDNA origin, for instance obtained by preparing a genomic or cDNA library and screening for DNA sequences coding for all or part of the polypeptide of the invention by hybridization using synthetic oligonucleotide probes in accordance with standard techniques (Sambrook et al., Molecular Cloning: A Laboratorv Manual, Cold Spring Harbor, 1989). Finally, the DNA construct may be of mixed synthetic and genomic, mixed synthetic and cDNA or mixed genomic and cDNA origin prepared by annealing fragments of synthetic, genomic or cDNA origin, as appropriate, the fragments corresponding to various parts of the entire DNA construct, in accordance with standard techniques.

Transformants according to the present invention can be obtained by introducing such a vector DNA, e.g. plasmid DNA, into a host and selecting transformants which express the POI or the host cell metabolite with high yields. Host cells are treated to enable them to incorporate foreign DNA by methods conventionally used for transformation of eukaryotic cells, such as the electric pulse method, the protoplast method, the lithium acetate method, and modified methods thereof. *P. pastoris* is preferably transformed by electroporation.

In another preferred embodiment, the yeast expression vector is able to stably integrate in the yeast genome, e. g. by homologous recombination.

A transformant host cell according to the invention obtained by transforming the cell with the regulatory elements according to the invention and/or the POI genes may preferably first be cultivated at conditions to grow efficiently to a large cell number without the burden of expressing a heterologous protein. When the cell line is prepared for the POI expression, cultivation techniques are chosen to produce the expression product.

When the transformant is grown with an inductive stimulus, a promoter may be activated to direct transcription of the gene under its control, and the POI is expressed. Under growth conditions with an inductive stimulus, it usually grows slower than under normal conditions, but because it has already grown to a large cell number in the previous stage, the culture system as a whole produces a large amount of the heterologous protein. An inductive stimulus is preferably heat, or addition of cadmium, copper, an osmotic pressure increasing agent, hydrogen peroxide, ethanol, methanol, methylamine or the like.

It is preferred to cultivate the host cell line according to the invention in a bioreactor under growth conditions to obtain a cell density of at least 1 g/L cell dry weight, more preferably at least 10 g/L cell dry weight, preferably at least 50 g/L cell dry weight. It is advantageous to provide for such yields of biomass production on a pilot or industrial scale.

The POI is preferably expressed employing conditions to produce yields of at least 1 mg/L, preferably at least 10 mg/L, preferably at least 100 mg/L, most preferred at least 1 g/L.

The host cell according to the invention is preferably tested for its expression capacity or yield by the following test: ELISA, activity assay, HPLC, or other suitable tests.

Preferred fermentation techniques are batch, fed batch or continuous cultivation.

Preferably the yeast is cultivated in a mineral medium with a suitable carbon source, thereby further simplifying the isolation process significantly. An example of a preferred mineral medium is one containing an utilizable carbon source (e.g. glucose, glycerol or methanol), salts containing the macro elements (potassium, magnesium, calcium, ammonium, chloride, sulphate, phosphate) and trace elements (copper, iodide, manganese, molybdate, cobalt, zinc, and iron salts, and boric acid), and optionally vitamins or amino acids, e.g. to complement auxotrophies.

The transformed cells are cultivated under conditions suitable to effect expression of the desired POI, which can be purified from the cells or culture medium, depending on the nature of the expression system and the expressed protein, e.g. whether the protein is fused to a signal peptide and whether the protein is soluble or membrane-bound. As will be understood by the skilled artisan, cultivation conditions will vary according to factors that include the type of host cell and particular expression vector employed.

If the desired compound is secreted from the cells, it can be isolated and purified from the culture medium using state of the art techniques. Secretion of the recombinant expression products from the yeast cells is generally advantageous for reasons that include facilitating the purification process, since the products are recovered from the culture supernatant rather than from the complex mixture of proteins that results when yeast cells are disrupted to release intracellular proteins.

The cultured transformant cells may also be ruptured sonically or mechanically, enzymatically or chemically to obtain a cell extract containing the desired POI, from which the POI is isolated and purified.

As isolation and purification methods for obtaining a recombinant polypeptide or protein product, methods, such as methods utilizing difference in solubility, such as salting out and solvent precipitation, methods utilizing difference in molecular weight, such as ultrafiltration and gel electrophoresis, methods utilizing difference in electric charge, such as ion-exchange chromatography, methods utilizing specific affinity, such as affinity chromatography, methods utilizing difference in hydrophobicity, such as reverse phase high performance liquid chromatography, and methods utilizing difference in isoelectric point, such as isoelectric focusing may be used. Specific purification steps are preferably employed to separate any Epx1 polypeptide that is co-expressed and would contaminate the POI preparation.

In some cases it is preferred to separate the POI or host cell metabolite from the Epx1 polypeptide which may be present in the the cell culture, thus to obtain the POI, any metabolite and optionally the Epx1 polypeptide in the purified form.

The highly purified product is essentially free from contaminating proteins, and preferably has a purity of at least 90%, more preferred at least 95%, or even at least 98%, up to 100%. The purified products may be obtained by purification of the cell culture supernatant or else from cellular debris. By means of the method according to the invention, it was the first time possible to obtain an Epx1 polypeptide in the highly purified form.

To avoid contamination by the Epx1 polypeptide that could be expressed concomitantly with a POI by a *P. pastoris* host, it is preferred to provide for a knock-out host cell, which has a non-functional disruption of either the Epx1 regulatory elements or the Epx1 gene, to down-modulate or eliminate the Epx1 expression. The Epx1 knock-out *P. pastoris* may be produced by a method for knocking down, partially or completely, the Epx1 gene. The knockout host cell line according to the invention is particularly suitable for industrial production processes, wherein the product is obtained in the absence of any Epx1 contamination, thus immediately available in a purified form. In those instances in which gene function or expression is downmodulated or eliminated, the resulting cell or organism can also be referred to as a knock-out. One embodiment of the method of producing knockdown cells and organisms comprises introducing into a cell or organism in which Epx1 is to be knocked down, RNA that targets the Epx1 gene or its regulatory sequences and maintaining the resulting cell or organism under conditions under which antisense RNA occurs, resulting in degradation or inactivation of the Epx1 mRNA or its regulatory sequences, thereby producing knockdown cells or organisms.

The isolated and purified POI can be identified by conventional methods such as Western blotting or assay of its activity. The structure of the purified compound can be defined by amino acid analysis, amino-terminal analysis, primary structure analysis, and the like. It is preferred that the compound is obtainable in large amounts and in a high purity level, thus meeting the necessary requirements for being used as an active ingredient in pharmaceutical compositions.

The preferred host cell line according to the invention maintains the integration of the regulatory sequences according to the invention and POI gene, and the expression level remains high, e.g. at least at a µg level, even after about 20 generations of cultivation, preferably at least 30 generations, more preferably at least 40 generations, most preferred of at least 50 generations. The recombinant host cell is surprisingly stable, which is a great advantage when used for industrial scale protein production.

The present invention is described in further detail in the following examples, which are not in any way intended to limit the scope of the invention as claimed.

### Examples

### Example 1: Identification of the P. pastoris protein Epx1

### 1a): Cultivation of recombinant P. pastoris

In order to produce a 2F5 Fab fragment of human antibody (Gasser et al 2006. Biotechnol. Bioeng. 94, 353-361) a *P. pastoris* strain X-33 (Invitrogen, wild type strain) expressing extracellularly the protein of interest under control of the GAP promoter was cultivated in a bioreactor.

For the generation of an expression strain for the Fab fragment, the entire light chain genes (vL and cL) and the vH and cH1 region of the heavy chain genes were amplified by PCR. The light chain (LC) fragment was ligated into a modified version of pGAPZalfaA, where the Avrll restriction site was changed into Ndel by site directed mutagenesis to allow subsequent linearization of the plasmids containing two cassettes. The heavy chain (HC) fragments were inserted into the original version of pGAPZalfaA. Plasmids combining the expression cassettes for both Fab chains on one vector were produced by double digestion of the light chain vector with Bgl II and BamHI, and subsequent insertion of these constructs into the unique Bgl II site of pGAPZalfaA already containing a single copy of the expression cassette of the heavy chain fragment. The plasmid was linearized with Avrll prior to electrotransformation into P. pastoris X-33.

The cultivation was carried out in a 5.0 L working volume bioreactor with a computer based process control. Culture temperature was controlled at 25°C, pH was controlled at 5.0 with addition of 25% ammonium hydroxide and the dissolved oxygen concentration was maintained above 20% saturation by controlling the stirrer speed between 600 and 1200 rpm, whereas the airflow was kept constant at 120 L h⁻¹. A shake flask containing 100 mL of YPG medium (per liter: 10 g yeast extract, 10 g peptone, 10 g glycerol) was inoculated with one cryovial from the *P. pastoris* cell bank, and incubated at 28°C for approximately 24 hours and agitated at 180 rpm. This culture was used to inoculate the starting volume of 1.75 L batch medium to a starting optical density (OD₆₀₀) of 1.0. After approximately 24 hours the batch was finished and a fed batch was followed by starting a glucose feed with a constant feed rate of F = 13.0 g h⁻¹. The cultivation was terminated after. t = 96 h.

The culture broth was harvested and centrifuged at 2500 g, 15 minutes at 4°C. The supernatant was 0.2 µm filtrated (Sartobran 300, Sartorius) and stored at 4°C for further processing.

Batch medium contained per liter:
2.0 g citric acid, 12.4 g (NH₄)₂HPO₄, 0.022 g CaCl₂·2H₂O, 0.9 g KCl, 0.5 g MgSO₄·7H₂O, 40 g glycerol, 4.6 ml PTM₁ trace salts stock solution. The pH was set to 5.0 with 25 % HCl.

Glucose fed batch solution contained per liter:
550 g glucose·1H₂O, 10 g KCl, 6.45 g MgSO₄·7H₂O, 0.35 g CaCl₂·2H₂O and 12 ml PTM₁ trace salts stock solution.

PTM1 trace salts stock solution contained per liter:
6.0 g CuSO₄·5H₂O, 0.08 g Nal, 3.0 g MnSO₄·H₂O, 0.2 g Na₂MoO₄·2H₂O, 0.02 g H₃BO₃, 0.5 g CoCl₂, 20.0 g ZnCl₂, 65.0 g FeSO₄·7H₂O, 0.2 g biotin and 5.0 ml H₂SO₄ (95 %-98 %).

### 1b): Concentration of P. pastoris supernatant

2 L of supernatant was concentrated by ultrafiltration using a Millipore Labscale TFF system (Millipore Corporation, Bedford, MA), which was equipped with Pellicon XL filter membranes (filtration area 50 cm² per unit, 10 kDa cut off, Millipore). The whole system was placed into a MiniColdLab (LKB 2023, Bromma, Sweden) and tempered at 4 °C. The system was operated at a permeate flow of 10 mL min⁻¹. Feed pressure was 20 psi and retentate pressure 10 psi. The supernatant was concentrated to a volume of 100 mL (1:20) and diafiltered against 600 mL of a 10 mM Tris/HCl buffer, pH 7.5. The conductivity of the diafiltrate was 1.2 mS cm⁻¹.

### 1c): Ion exchange chromatography (IEC)

The IEC of the concentrated supernatant was performed at a Biologic DUO FLOW (Biorad) chromatography station, using a SP Sepharose Fast Flow, Tricon 5.50, 1 mL gel column (GE Healthcare). The buffers used were buffer A (10mM Tris pH 7.5) and buffer B (10mM Tris 1mM salt). The zero baseline was set at 100 % buffer A , 0 % buffer B, volume 1.00 mL and flow 1.00 mL min⁻¹. At this point 30 mL sample were loaded and injected by the auto inject valve at flow 0.50 mL min⁻¹. The isocratic flow was done at 100 % buffer A, 0 % buffer B, volume 3.00 mL and flow 0.50 mL min⁻¹. The first of two elution steps was realized by a linear gradient at 100 % → 70 % buffer A, 0 % → 30% buffer B, volume 10.00 mL and flow 0.50 mL min⁻¹, the second one at a linear gradient at 70 % → 0 % buffer A, 30 % → 100 % buffer B, volume 10.00 mL and flow 0.50 mL/min. The fractions were collected by a fraction collector (BioFrac) and stored at 4°C for further purification. The chromatogram showed a double peak from 38 to 45 mL. SDS Page analysis of the elution fractions 27 to 31 showed a strong protein band at approximately 45 kDa (Fab fragment), whereas fraction 32 to 36 showed a strong protein band at 60-70 kDa, which is a unknown host cell protein (Epx1 protein)

### 1d): Size exclusion chromatography (SEC)

For further polishing the fractions 28 -32 from Ion Exchange Chromatography were pooled for preparative Gel Chromatography using a HiLoad™ Prep Grade Column (16/60: 120 mL , GE Healthcare) packed with Superdex™ 75. The column was equilibrated with PBS using a flowrate of 30 cm h⁻¹ (= 1 mL min⁻¹). 1 ml sample was loaded and collected in 2ml-fractions.

The chromatogram of the SEC showed three separated peaks. SDS Page analysis of the fractions identified the first small peak as a 25 kDa protein (the LC and HC monomers of the Fab fragment), the second clear peak as a band of 45 - 50 kDa (dimer of the Fab fragment) and the third peak consisting of a protein with 60-70 kDa (host cell protein, putative Epx1).

### Example 2: Identification of P. pastoris Epx1 protein by protein sequencing

### 2a) Identification of P. pastoris Epx1 by N-terminal protein sequencing:

The purified protein was subjected to SDS-PAGE separation and Western Blotting using NuPAGE gels (Invitrogen) according to supplier's instructions. The proteins were stained with Coomassie Brillant Blue, and the protein band corresponding to a molecular weight of approx. 65 kDa was analysed by N-terminal Edman sequencing. This resulted the N-terminal peptide LKPGESI.

### 2b) Identification of the P. pastoris Epx1 by LC-MS/MS:

The protein band at approx. 65 kDa was picked from the CBB stained gel. After tryptic digest, samples were analyzed by reversed-phase chromatography (UltiMate 3000 Capillary LC-system, Dionex) coupled with ESI MS/MS analysis (Q-TOF Ultima Global, Waters). The obtained mass spectra were subsequently analysed using X!Tandem 2008.12.01 (http://www.thegpm.org/tandem/). The identified protein had to meet the following criteria: protein score e-value ≤ 10⁻⁵ with at least 2 peptides per protein. The identified peptides were: (i) ECSTDRYYIICEYAPR; (ii)YYIICEYAPR; (iii) GNIVSAGYFEDNVLPPV. *De novo* sequencing and BLAST searches against the *P. pastoris* sequence information were performed, and yielded the protein of SEQ ID No. 1. The separated protein forms a probably disulfide bridge linked dimer, as the molecular weight of the identified sequence is 31.7 kDa.

SEQ ID No. 1: Epx1 protein sequence of *P. pastoris* X-33. Identified peptides are highlighted in bold. The protein sequence of the mature Epx1 protein starts with amino acid position 58.

MKLSTNLILAIAAASAVVSAAPVAPAEEAANHLHKRAYYTDTTKTHTFTEVVTVY
RTLKPGESIPTDSPSHGGKSTKKGKGSTTHSGAPGATSGAPTDDTTSTSGSVGLPTS
ATSVTSSTSSASTTSSGTSATSTGTGTSTSTSTGTGTGTTGTGTTSSSTSSSATSTPT
GSIDAISQTLLDTHNDKRALHGVPDLTWSTELADYAQGYADSYTCGSSLEHTGGPYG
ENLASGYSPAGSVEAWYNEISDYDFSNPGYSAGTGHFTQVVWKSTTQLGCGYKECS
TDRYYIICEYAPRGNIVSAGYFEDNVLPPV

SEQ ID No. 2: Epx1 protein sequence of *P. pastoris* DSMZ 70382. Identified peptides are highlighted in bold: (i) SAGTGHFTQVVWK; (ii) STTQLGCGYK; (iii) YYIICEYAPR; (iv) STTQLGCGYKECGTNR; (v) GNIVSAGYFEDNVLPLV. The protein sequence of the mature Epx1 protein starts with amino acid position 58.

MKFSTNLILAIAAASTVVSAAPVAPAEEAANHLHKRAYYTDTTKTHTFTEVVTVY
RTLKPGESIPTGSPSHGGKGSKTGKGKGSTTDAGVPGTTSVAPTADSTSASGSIGLP
TSASSATSATSSASTTTSGTSTSSTGTGTTTGATTGTGTSSTGTTSSSTSSSATSTPT
GSLDAISQSLLDTHNEKRALHGVSDLTWSTELADYAQGYADSFTCGSSLVHTGGPYG
ENLASGYSPAGSVEAWYNEISDYDFSNPGYSAGTGHFTQVVWKSTTQLGCGYKEC
GTNRYYIICEYAPRGNIVSAGYFEDNVLPLV

The signal peptide sequence (SEQ ID No 3) was identified using the SignaIP 3.0 prediction tool (http://www.cbs.dtu.dk/services/SignaIP/). The most likely cleavage site for the signal peptidase is between pos. 20 and 21 (VSA-AP).

The leader peptide (SEQ ID No 4) is determined by the native N-terminus of the secreted protein as identified by Edman sequencing, and the signal peptidase cleavage site.

### Example 3: Construction of an expression vector containing the Epx1

### precursing sequence (signal sequence and leader sequence)

To generate an expression vector using the secretion leader sequence of the *P. pastoris* Epx1 protein for secretion of a POI, the respective sequence (SEQ ID No.5) is cloned in frame with the P_{GAP} promoter (glycerol aldehyde phosphate dehydrogenase promoter) of the pPM2_pGAP expression vector. The pPM2d_pGAP expression vector is a derivative of the pPuzzle_zeoR_AOXTT vector backbone described in WO2008/128701A2, consisting of the pUC19 bacterial origin of replication, the *P. pastoris* glycerol aldehyde phosphate dehydrogenase promoter, the *S. cerevisiae* CYC1 transcription terminator, and the Zeocin antibiotic resistance cassette.

The Epx1 secretion leader sequence is amplified by PCR from *P. pastoris* X-33 using the oligonucleotide primers (Table 1).

**Table 1: Oligonucleotide primer for PCR amplification of the precursing sequence of the P. pastoris Epx1 protein**

| | |
|---|---|
| EpxL-SbfI; Tm=64°C | 5'-TATACCTGCAGGATGAAGTTCTCTACCAATTTGATC-3' |
| | (SEQ ID No. 8) |
| EpxL-NsiI; Tm=64°C | 5'-GAAGATGCATCGTACGGTAGACAGTGACAAC-3' |
| | (SEQ ID No. 9) |

Subsequently, the PCR product (189 bp) is digested by the restriction enzymes SbfI and NsiI; and ligated into a pPM2d_pGAP vector that has been linearized by SbfI and treated with calf intestine phosphatase (CIP). As NsiI and SbfI produce overlapping ends, the resulting construct pPM2d_pGAPxL has a single SbfI site directly before the start codon of the Epx1-leader sequence. Correct integration is verified by digestion of the resulting plasmids with SbfI and AscI.

### Example 4: Construction of an expression vector containing the Epx1 promoter

### sequence

To generate an expression vector using the regulatory sequence of the *P. pastoris* Epx1 gene to drive expression of a POI, the respective sequence (SEQ ID No. 7) is exchanged against the P_{GAP} promoter of the pPM2d_pGAP expression vector. The Epx1 promoter sequence is obtained from *P. pastoris* X-33 by amplifying 1000bp of the 5'-non coding region of the respective gene using the oligonucleotide primers of SEQ ID No. 10 and SEQ ID No. 11 (Table 2).

**Table 2: Oligonucleotide primer for PCR amplification of the promoter sequence of the P. pastoris Epx1 gene**

| | |
|---|---|
| pEPX-MreI; Tm=64°C | 5'-ATTACGCCGGCGGCATCAAGGGTTCTGGATCTC-3' |
| | (SEQ ID No. 10) |
| pEPX-SbfI; Tm=62°C | 5'-GATCCCTGCAGGTAGAATAGTGAACGTGTTTTAAAG-3' |
| | (SEQ ID No. 11) |

Both the PCR product (1000 bp) and the pPM2d_pGAP vector are digested by the restriction enzymes MreI and SbfI. After treatment of the vector with CIP, the vector backbone and the Epx1 promoter fragment are ligated by alkaline phosphatase, generating the expression vector pPM2_pEPX.

### Example 5: Construction of an expression vector containing the Epx1 promoter and precursing sequence

To generate an expression vector using the regulatory sequence of the *P. pastoris* Epx1 gene to drive expression of a POI and the precursing sequence of the Epx1 protein for secretion of the POI, the respective promoter sequence (SEQ ID No. 7) is exchanged against the P_{GAP} promoter of the pPM2d_pGAPxL expression vector (described in Example 3).

Both the promoter region (1000 bp) and the pPM2d_pGAPxL vector are digested by the restriction enzymes MreI and SbfI. After treatment of the vector with CIP, the vector backbone and the pEPX1 fragment are ligated by alkaline phosphatase, generating the expression vector pPM2d_pEPxL.

### Example 6: Construction of a P. pastoris host cell line for the expression of recombinant trypsinogen

### 6a) Construction of a P. pastoris strain overexpressing recombinant porcine trypsinogen using the pPM2 pGAPxL vector.

For expression of recombinant porcine trypsinogen (rpTRP) a codon-optimized artificial gene was synthesized (Geneart Germany). Sites for the restriction enzymes

Pfl23II and SfiI, flanking the open reading frame were added during PCR amplification using the delivered plasmid as template and the oligonucleotide primer of SEQ ID No. 12 and SEQ ID No. 13 (Table 3). The PCR product was digested with Pfl23II and SfiI, and cloned into a Pfl23II, SfiI and CIP treated plasmid pPM2_pGAPxL (example 3). The ligated plasmid is transformed into *E. coli* TOP10 (Invitrogen) and plated on Zeocin containing LB-agar. Restriction endonuclease analysis was performed to confirm the correct identity of the plasmid pPM2_pGAPxL_rpTRP.

**Table 3: Oligonucleotide primer for PCR amplification of the promoter sequence of the gene for porcine trypsinogen**

| | |
|---|---|
| rptrp-Pfl23II; | 5'- ATACCGTACGTTCACTGACGACGACGACAAG -3' |
| Tm=64°C | (SEQ ID No. 12) |
| rptrp-SfiI; Tm=62°C | 5'-TTTTGGCCGAGGCGGCCTTTCAGTTAGCAGCGATAGTTTG -3' |
| | (SEQ ID No. 13) |

### 6b) Construction of a P. pastoris strain overexpressing recombinant porcine trypsinogen using the pPM2 pEPX vector.

The gene for porcine trypsinogen fused to the *S. cerevisiae* alfa-mating factor sequence as secretion leader is obtained from the plasmid described in Baumann et al. 2008. Biotech. Bioeng., 100(1):177-183, by restriction digest with SbfI and SfiI. This fragment is ligated into the pPM2_pEPX plasmid (example 4) that has also been digested with SbfI and SfiI, and treated with calf intestine phosphatase.

The ligated plasmid is transformed into *E. coli* TOP10 (Invitrogen) and plated on Zeocin containing LB-agar. Restriction endonuclease analysis is performed to confirm the correct identity of the plasmid pPM2_pEPX_alfarpTRP.

### 6c) Construction of a P. pastoris strain overexpressing recombinant porcine trypsinogen using the pPM2 pEpxL vector.

The gene encoding rpTRP is obtained from pPM2_pGAPxL_rpTRP (example 6a) by restriction digest with Pfl23II and SfiI, and cloned into a Pf123II, SfiI and CIP treated plasmid pPM2_pEPxL (example 5). The ligated plasmid is transformed into *E. coli* TOP10 (Invitrogen) and plated on Zeocin containing LB-agar. Restriction endonuclease analysis is performed to confirm the correct identity of the plasmid pPM2_pEPxL_rpTRP.

All plasmids are linearized in the respective promoter region prior to electrotransformation into *P. pastoris*. Positive transformants are selected on YPD agar plates containing yeast extract (10 g/L), peptone (20 g/L), glucose (20 g/L) and Zeocin.

### 6d) Culturing transformed P. pastoris strains in shake flask cultures

5 ml YP-medium (10 g/L yeast extract, 20 g/L peptone) containing 10 g/L glycerol are inoculated with a single colony of *P. pastoris* strains from Example 5 a-c and grown overnight at 28°C. Aliquots of these cultures (corresponding to a final OD600 of 0.1) are transferred to 10 mL of expression culture medium (per liter: 10 g yeast extract, 10 g pea-peptone, 10.2 g (NH₄)₂PO₄, 1.24 g KCl, 0.1 g CaCl₂, pH 5.0 adjusted with HCl) supplemented with 20 g/L glucose and incubated for 48 h at 28°C at 170 rpm in 100 ml Erlenmeyer flasks. Glucose (10 g/L) is added repeatedly every 12 h, before cells are harvested by centrifugation at 2500xg for 10 min at room temperature and prepared for analysis. Biomass is determined by measuring the cell weight of 1 mL cell suspension, while trypsinogen in the culture supernatant is quantified as described in Example 6e.

### 6e) Quantification of rhTRP

Desalting of *P. pastoris* culture supernatant is done using small pre-packed size exclusions chromatography columns (Disposable PD-10 Desalting Columns 17-0851-01; GE Healthcare). 2.5 mL of supernatant are applied to the column and eluted with 3.5mL of elution buffer (1 mM HCl). After elution 70µL of 2M CaCl₂ solution was added. To convert inactive trypsinogen to the active trypsin 300µL of the desalted supernatant (+CaCl₂) are mixed with 690µL of activation buffer (50mM TRIS/HCl pH 8.6; 40mM CaCl₂ and 0.15 g/L Enterokinase, Sigma; E0632) and incubated for two hours at 37°C. 165µL of the activation mixture is mixed with 1000µL of TAME-solution, containing 446 mg/L *N*_{α}-*p*-Tosyl-L-arginine-methyl-ester-hydrochloride (TAME; Sigma; T4626) dissolved in dilution buffer (50 mM TRIS/HCl pH 8.1; 40mM CaCl₂) and an absorption kinetic at 247nm is measured in a spectrophotometer over a time period of 5 min. If necessary, activated trypsin solution is diluted with dilution buffer to hit the linear range (ΔA₂₄₇/min < 0.3) of this method. A trypsin concentration of 1g/L corresponds to ΔA₂₄₇/min= 0.101.

### 6f) Method of producing recombinant trypsinogen in a bioreactor

Fed batch cultivations are carried out in a 3.5 L working volume bioreactor (Infors, Switzerland) with a computer based process control. Fermentation temperature is controlled at 25°C, pH is controlled at 5.0 with addition of 25% ammonium hydroxide and the dissolved oxygen concentration is maintained above 20% saturation by controlling the stirrer speed between 600 and 1200 rpm, whereas the airflow is kept constant at 100 L h⁻¹.

The media are as follows:
Batch medium contains per liter:
   2.0 g citric acid, 12.4 g (NH4)₂HPO₄, 0.022 g CaCl₂·2H₂O, 0.9 g KCl, 0.5 g MgSO₄·7H₂O, 40 g glycerol, 4.6 ml PTM1 trace salts stock solution. The pH is set to 5.0 with 25% HCl.

Glucose fed batch solution contains per liter:
550 g glucose·1H₂O, 10 g KCl, 6.45 g MgSO₄·7H₂O, 0.35 g CaCl₂·2H₂O and 12 ml PTM1 trace salts stock solution. PTM1 composition is given in Example 1.

After approximately 27 h, the batch is finished and the glucose fed batch with a constant feed rate of 16 g h⁻¹ is started. The fermentations are terminated at approximately 100 h. Samples are taken frequently for the determination of biomass and the quantification of trypsinogen (as described in Example 6d).

### Example 7: Generation of a P. pastoris Epx1 knock-out strain

*Pichia pastoris* knock-out strains are generated by making a non-functional disruption of a specific gene (Marx et al. 2006, FEMS Microbiol Lett 264:40-47). A disruption cassette for the Epx1 gene with 300-800 bps flanking regions is generated by PCR. Subsequently, the region around the start codon (approx 200-400 bps up-and downstream of the ATG) is exchanged for the resistance marker cassette. The cloning of the 3'and 5'-flanking regions is done separately in a vector already containing the resistance marker, as described in Marx et al. 2006.

The disruption cassette is introduced into the genome of *P. pastoris* by electroporation, and clones are selected on YP plates containing 25 µg/mL Zeocin. Positively growing clones are then analyzed for correct integration of the disruption cassette by PCR (using primers outside of the disruption cassette) on a genomic level. Disruption clones are then cultivated as described in Example 6d and analyzed for their growth behavior. Additionally, the culture supernatant is assayed for the lack of appearance of the Epx1 protein band by SDS-PAGE (Example 2).

### Example 8: Expression of recombinant trypsinogen in a P. pastoris Epx1 knock-out strain

The *P. pastoris* Epx1 knock-out strain Δepx1 is analysed for production of recombinant trypsinogen as described in the previous examples. After transformation of Δepx1 with either of the vectors constructed in Examples 3-5, positive transformants are selected due to their growth on Zeocin containing YPD-agar plates and cultivated as in Example 6d. Fed batch cultivation of the trypsinogen expressing Δepx1 strain is described in Example 6f.

Quantification of recombinant trypsinogen is performed as in Example 6e, and the quality of the supernatant is determined by SDS-PAGE as in Examples 2 and 7.
SEQ ID No 3 (signal peptide):
   MKFSTNLILAIAAASTVVSA
SEQ ID No 4 (leader peptide):
   APVAPAEEAANHLHKRAYYTDTTKTHTFTEVVTVYRT

SEQ ID No 5 (leader_171 nt):
ATGAAGTTCTCTACCAATTTGATCTTAGCTATTGCAGCAGCATCCACTGTTG
TCTCAGCTGCTCCAGTTGCTCCAGCCGAAGAGGCAGCAAACCACTTGCACAAGC
GTGCTTATTACACCGACACAACCAAGACTCACACTTTCACTGAGGTTGTCACTGTC
TACCGTACG

SEQ ID No 6 (leader_aa):

MKFSTNLILAIAAASTVVSAAPVAPAEEAANHLHKRAYYTDTTKTHTFTEVVTVY
RT

SEQ ID No 7 (promoter_1000 nt):
GCATCAAGGGTTCTGGATCTCTCGTGGATACATTCCGCGATGGGTTGCTCT
ATTCAAAGGAACTTGCTGACCTGAAAAGATTTGAATATGCAGAAGATCTGGAGTTC
TATGACGCTGAAGAAGAGCTGAACTTCAAGCAAGGGATTGCCGGCAACGGA
AAAACTAGCAAACACCAGAGACTCTTATCCCAGGATATCTCCAATAATTCCGTGAA
ACTAACCCCAGTAAACGGTATCAACAGTGATTCGTATGTATCGGTGAATACATAAT
TCCCTCCTGTTTAGGCAAATGTCATGCTTCAAATATAATACCCACGTTTGTTATTGT
TACCCCTATCGTTTTGTCTAGAACACTATTTATCGCACCTAACTATGCCTAGAACA
CCGAAGATCCCTCTCATTGGCTCGAGACATGGGGATATAGAATGGATCCTGAGAG
AAAATTGTTTACATTTGGAATTGAGTTAGCCCCCTGTGATTATGACAACATGCATG
CTGATGCATTCAATGCATGTGCATTCTGATATGAGAATTTCATCTACTAGATCGCA
ACGAAAAGTGGAAAAAAAAAGTAGGCCCATCCCTCTGGTAAAGAGAGTTGAATCA
ACAAAGCTCAGGCATGCTAGTCTATTGCGCATCCGCTATCATTTCGCCGTTTCAAT
GTACCTTTTGTTTGTGATAATTTCTCGACTCATCACGTTATTCCAAAATTTCACTCG
AAACAATTAGTCGTGCAGATACATTTCCAGTACACCTGAAATCAGTCTTCTTCTCT
ACTATTAAGAAACGCGTTATTTACGTCTCCCCCTTTTTGGAACCCTGGCCCCCAAG
GTTTGTTAGAGAAGCTATAAGACTCCTGTGGTTCCCTGAAATCGTGTTGTTTGTGA
TTCTTTTTGATACATCAAGTCTTTTTCGATAAAACCAACGAAGATACTCTTGAGTCG
TTTTCTGCAAATCCCAACTACCTGTCACTCCTTTAAAACACGTTCACTATTCTA

## Claims

1. Regulatory sequence of a *Pichia pastoris* derived expression system capable of expressing the Epx1 protein that comprises the sequence of the mature protein of SEQ ID No 1 or 2, which regulatory sequence is selected from the group consisting of a promoter, a signal sequence, a leader sequence and combinations thereof.

2. Sequence according to claim 1, which comprises a combination of said signal sequence with said promoter and/or leader sequence.

3. Sequence according to claim 1 or 2, which comprises a combination of said promoter with said signal sequence and/or said leader sequence.

4. Sequence according to any of claims 1 to 3, which comprises a promoter consisting of a base sequence of at least 150bp having at least 70% homology with the base sequence of SEQ ID NO.: 7.

5. Sequence according to any of claims 1 to 4, which comprises a signal sequence consisting of a base sequence encoding a peptide of at least 10 peptides, having at least 70% homology with the amino acid sequence of SEQ ID NO.: 3.

6. Sequence according to any of claims 1 to 5, which comprises a leader sequence consisting of a base sequence encoding a peptide of at least 10 peptides, having at least 70% homology with the amino acid sequence of SEQ ID NO.: 4.

7. Sequence according to any of claims 1 to 6, comprising a cleavage site for the signal peptidase between a signal sequence and a leader sequence and/or a cleavage site for a peptidase between a leader sequence and the N terminus of a POI.

8. Vector comprising a regulatory sequence according to any of claims 1 to 7.

9. Vector according to claim 8, further comprising a gene encoding a protein of interest (POI), optionally with at least one processing site that provides for protease cleavage of said POI.

10. Recombinant eukaryotic host cell comprising a regulatory sequence according to any of claims 1 to 7 or a vector according to any of claims 8 or 9.

11. Host cell according to claim 10, which is a fungal cell, preferably a yeast cell, such as a cell of the *Pichia* genus, in particular a cell of a strain of *P. pastoris*, or a higher eukaryotic cell, preferably a mammalian or a plant cell.

12. A method of producing a POI in a eukaryotic cell, comprising:
- providing a host cell according to any of claims 10 or 11 containing a POI gene, and
- cultivating said host cell to express said POI.

13. A method according to claim 12, wherein said POI is a polypeptide selected from the group consisting of serum proteins, such as an immunoglobulin or serum albumin, enzymes, hormones, signalling molecules, matrix proteins, fragments or derivatives thereof, optionally obtained as a secreted product.

14. A method according to claim 12 or 13, wherein said POI mediates the production of a host cell metabolite, optionally obtained as a secreted product.

15. *P. pastoris* host cell comprising a non-functional disruption of
- a regulatory sequence according to any of claims 1 to 7, and/or
- a sequence encoding the Epx1 protein.

16. Purified Epx1 protein comprising the amino acid sequence of SEQ ID No 1.


SEQUENCE LISTING

<110>  Universität für Bodenkultur Wien

<120>  Expression sequences

<130>  BK2/EP

<160>  13

<170>  PatentIn version 3.3

<210>  1
<211>  313
<212>  PRT
<213>  Pichia pastoris X-33

<400>  1

Met Lys Leu Ser Thr Asn Leu Ile Leu Ala Ile Ala Ala Ala Ser Ala
1               5                   10                  15

Val Val Ser Ala Ala Pro Val Ala Pro Ala Glu Glu Ala Ala Asn His
            20                  25                  30

Leu His Lys Arg Ala Tyr Tyr Thr Asp Thr Thr Lys Thr His Thr Phe
        35                  40                  45

Thr Glu Val Val Thr Val Tyr Arg Thr Leu Lys Pro Gly Glu Ser Ile
    50                  55                  60

Pro Thr Asp Ser Pro Ser His Gly Gly Lys Ser Thr Lys Lys Gly Lys
65                  70                  75                  80

Gly Ser Thr Thr His Ser Gly Ala Pro Gly Ala Thr Ser Gly Ala Pro
                85                  90                  95

Thr Asp Asp Thr Thr Ser Thr Ser Gly Ser Val Gly Leu Pro Thr Ser
            100                 105                 110

Ala Thr Ser Val Thr Ser Ser Thr Ser Ser Ala Ser Thr Thr Ser Ser
        115                 120                 125

Gly Thr Ser Ala Thr Ser Thr Gly Thr Gly Thr Ser Thr Ser Thr Ser
    130                 135                 140

Thr Gly Thr Gly Thr Gly Thr Thr Gly Thr Gly Thr Thr Ser Ser Ser
145                 150                 155                 160

Thr Ser Ser Ser Ala Thr Ser Thr Pro Thr Gly Ser Ile Asp Ala Ile
                165                 170                 175

Ser Gln Thr Leu Leu Asp Thr His Asn Asp Lys Arg Ala Leu His Gly
            180                 185                 190

Val Pro Asp Leu Thr Trp Ser Thr Glu Leu Ala Asp Tyr Ala Gln Gly
        195                 200                 205

Tyr Ala Asp Ser Tyr Thr Cys Gly Ser Ser Leu Glu His Thr Gly Gly
    210                 215                 220

Pro Tyr Gly Glu Asn Leu Ala Ser Gly Tyr Ser Pro Ala Gly Ser Val
225                 230                 235                 240

Glu Ala Trp Tyr Asn Glu Ile Ser Asp Tyr Asp Phe Ser Asn Pro Gly
                245                 250                 255

Tyr Ser Ala Gly Thr Gly His Phe Thr Gln Val Val Trp Lys Ser Thr
            260                 265                 270

Thr Gln Leu Gly Cys Gly Tyr Lys Glu Cys Ser Thr Asp Arg Tyr Tyr
        275                 280                 285

Ile Ile Cys Glu Tyr Ala Pro Arg Gly Asn Ile Val Ser Ala Gly Tyr
    290                 295                 300

Phe Glu Asp Asn Val Leu Pro Pro Val
305                 310

<210>  2
<211>  313
<212>  PRT
<213>  Pichia pastoris DSMZ 70382

<400>  2

Met Lys Phe Ser Thr Asn Leu Ile Leu Ala Ile Ala Ala Ala Ser Thr
1               5                   10                  15

Val Val Ser Ala Ala Pro Val Ala Pro Ala Glu Glu Ala Ala Asn His
            20                  25                  30

Leu His Lys Arg Ala Tyr Tyr Thr Asp Thr Thr Lys Thr His Thr Phe
        35                  40                  45

Thr Glu Val Val Thr Val Tyr Arg Thr Leu Lys Pro Gly Glu Ser Ile
    50                  55                  60

Pro Thr Gly Ser Pro Ser His Gly Gly Lys Gly Ser Lys Thr Gly Lys
65                  70                  75                  80

Gly Lys Gly Ser Thr Thr Asp Ala Gly Val Pro Gly Thr Thr Ser Val
                85                  90                  95

Ala Pro Thr Ala Asp Ser Thr Ser Ala Ser Gly Ser Ile Gly Leu Pro
            100                 105                 110

Thr Ser Ala Ser Ser Ala Thr Ser Ala Thr Ser Ser Ala Ser Thr Thr
        115                 120                 125

Thr Ser Gly Thr Ser Thr Ser Ser Thr Gly Thr Gly Thr Thr Thr Gly
    130                 135                 140

Ala Thr Thr Gly Thr Gly Thr Ser Ser Thr Gly Thr Thr Ser Ser Ser
145                 150                 155                 160

Thr Ser Ser Ser Ala Thr Ser Thr Pro Thr Gly Ser Leu Asp Ala Ile
                165                 170                 175

Ser Gln Ser Leu Leu Asp Thr His Asn Glu Lys Arg Ala Leu His Gly
            180                 185                 190

Val Ser Asp Leu Thr Trp Ser Thr Glu Leu Ala Asp Tyr Ala Gln Gly
        195                 200                 205

Tyr Ala Asp Ser Phe Thr Cys Gly Ser Ser Leu Val His Thr Gly Gly
    210                 215                 220

Pro Tyr Gly Glu Asn Leu Ala Ser Gly Tyr Ser Pro Ala Gly Ser Val
225                 230                 235                 240

Glu Ala Trp Tyr Asn Glu Ile Ser Asp Tyr Asp Phe Ser Asn Pro Gly
                245                 250                 255

Tyr Ser Ala Gly Thr Gly His Phe Thr Gln Val Val Trp Lys Ser Thr
            260                 265                 270

Thr Gln Leu Gly Cys Gly Tyr Lys Glu Cys Gly Thr Asn Arg Tyr Tyr
        275                 280                 285

Ile Ile Cys Glu Tyr Ala Pro Arg Gly Asn Ile Val Ser Ala Gly Tyr
    290                 295                 300

Phe Glu Asp Asn Val Leu Pro Leu Val
305                 310

<210>  3
<211>  20
<212>  PRT
<213>  Pichia pastoris

<400>  3

Met Lys Phe Ser Thr Asn Leu Ile Leu Ala Ile Ala Ala Ala Ser Thr
1               5                   10                  15

Val Val Ser Ala
            20

<210>  4
<211>  37
<212>  PRT
<213>  Pichia pastoris

<400>  4

Ala Pro Val Ala Pro Ala Glu Glu Ala Ala Asn His Leu His Lys Arg
1               5                   10                  15

Ala Tyr Tyr Thr Asp Thr Thr Lys Thr His Thr Phe Thr Glu Val Val
            20                  25                  30

Thr Val Tyr Arg Thr
        35

<210>  5
<211>  171
<212>  DNA
<213>  Pichia pastoris

<400>  5
atgaagttct ctaccaattt gatcttagct attgcagcag catccactgt tgtctcagct      60

gctccagttg ctccagccga agaggcagca aaccacttgc acaagcgtgc ttattacacc     120

gacacaacca agactcacac tttcactgag gttgtcactg tctaccgtac g              171

<210>  6
<211>  57
<212>  PRT
<213>  Pichia pastoris

<400>  6

Met Lys Phe Ser Thr Asn Leu Ile Leu Ala Ile Ala Ala Ala Ser Thr
1               5                   10                  15

Val Val Ser Ala Ala Pro Val Ala Pro Ala Glu Glu Ala Ala Asn His
            20                  25                  30

Leu His Lys Arg Ala Tyr Tyr Thr Asp Thr Thr Lys Thr His Thr Phe
        35                  40                  45

Thr Glu Val Val Thr Val Tyr Arg Thr
    50                  55

<210>  7
<211>  1000
<212>  DNA
<213>  Pichia pastoris

<400>  7
gcatcaaggg ttctggatct ctcgtggata cattccgcga tgggttgctc tattcaaagg      60

aacttgctga cctgaaaaga tttgaatatg cagaagatct ggagttctat gacgctgaag     120

aagaagagct gaacttcaag caagggattg ccggcaacgg aaaaactagc aaacaccaga     180

gactcttatc ccaggatatc tccaataatt ccgtgaaact aaccccagta aacggtatca     240

acagtgattc gtatgtatcg gtgaatacat aattccctcc tgtttaggca aatgtcatgc     300

ttcaaatata atacccacgt ttgttattgt tacccctatc gttttgtcta gaacactatt     360

tatcgcacct aactatgcct agaacaccga agatccctct cattggctcg agacatgggg     420

atatagaatg gatcctgaga gaaaattgtt tacatttgga attgagttag ccccctgtga 480

ttatgacaac atgcatgctg atgcattcaa tgcatgtgca ttctgatatg agaatttcat 540

ctactagatc gcaacgaaaa gtggaaaaaa aaagtaggcc catccctctg gtaaagagag 600

ttgaatcaac aaagctcagg catgctagtc tattgcgcat ccgctatcat ttcgccgttt 660

caatgtacct tttgtttgtg ataatttctc gactcatcac gttattccaa aatttcactc 720

gaaacaatta gtcgtgcaga tacatttcca gtacacctga aatcagtctt cttctctact 780

attaagaaac gcgttattta cgtctccccc tttttggaac cctggccccc aaggtttgtt 840

agagaagcta taagactcct gtggttccct gaaatcgtgt tgtttgtgat tctttttgat 900

acatcaagtc tttttcgata aaaccaacga agatactctt gagtcgtttt ctgcaaatcc 960

caactacctg tcactccttt aaaacacgtt cactattcta 1000

<210> 8
<211> 36
<212> DNA
<213> artificial

<220>
<223> amplification primer EpxL-SbfI

<400> 8
tatacctgca ggatgaagtt ctctaccaat ttgatc 36

<210> 9
<211> 31
<212> DNA
<213> artificial

<220>
<223> amplification primer EpxL-NsiI

<400> 9
gaagatgcat cgtacggtag acagtgacaa c 31

<210> 10
<211> 33
<212> DNA
<213> artificial

<220>
<223> amplification primer pEPX-MreI

<400> 10
attacgccgg cggcatcaag ggttctggat ctc 33

<210> 11
<211> 36
<212> DNA
<213> artificial

<220>
<223> amplification primer pEPX-SbfI

<400> 11
gatccctgca ggtagaatag tgaacgtgtt ttaaag 36

<210>  12
<211>  31
<212>  DNA
<213>  artificial

<220>
<223>  amplification primer rptrp-Pfl23II

<400>  12
ataccgtacg ttcactgacg acgacgacaa g                                      31


<210>  13
<211>  40
<212>  DNA
<213>  artificial

<220>
<223>  amplification primer rptrp-SfiI

<400>  13
ttttggccga ggcggccttt cagttagcag cgatagtttg                             40